# EUROPÄISCHE PATENTSCHRIFT

(11) **EP 0 889 872 B1**
(45) Veröffentlichungstag und Bekanntmachung des Hinweises auf die Patenterteilung: **27.12.2000**
(21) Anmeldenummer: 97915375.6
(22) Anmeldetag: 17.03.1997
(51) Int. Cl.: C07C 67/26, C07C 69/24, C07C 69/52

(54) **VERFAHREN ZUR HERSTELLUNG ALKOXYLIERTER FETTSÄUREALKYLESTER**
PROCESS FOR PREPARING ALKOXYLATED FATTY ACID ALKYL ESTERS
PROCEDE DE PREPARATION D'ALKYLESTERS D'ACIDE GRAS ALCOXYLES

(30) Priorität: 25.03.1996 DE 19611508
(43) Veröffentlichungstag der Anmeldung: 13.01.1999
(73) Patentinhaber: Cognis Deutschland GmbH, 40589 Düsseldorf (DE)
(72) Erfinder: PI SUBIRANA, Rafael, E-08400 Granollers (ES); BIGORRA LLOSAS, Joaquim, E-08203 Sabadell (ES)
(86) Internationale Anmeldenummer: EP9701326
(87) Internationale Veröffentlichungsnummer: WO9735830

(56) Entgegenhaltungen:
- EP-A- 0 619 291

## Beschreibung

Die Erfindung betrifft ein Verfahren zur Alkoxylierung von Fettsäurealkylestern in Gegenwart eines homogenen Katalysator/Co-Katalysator-Systems.

### Stand der Technik

Alkoxylierte Alkylester, vorzugsweise sogenannte Methylesterethoxylate, stellen bekannte nichtionische Tenside dar, die wegen ihrer ausgezeichneten Waschleistung in letzter Zeit erheblich an Interesse gewonnen haben. Übersichten hierzu finden sich beispielsweise in **J.Am.Oil.Chem.Soc. 56, 873 (1979)** bzw. **J.Am.Oil.Chem.Soc. 72, 781 (1995)**.

Die Anlagerung von Alkylenoxiden an Verbindungen mit aciden Wasserstoffatomen, vorzugsweise an primäre Alkohole, gelingt in Gegenwart verschiedenster, in der Regel alkalischer Katalysatoren. Typische Beispiele sind Kaliumhydroxid oder Natriummethylat, die in Form alkoholischer Lösungen zugesetzt werden, oder aber heterogene Schichtverbindungen vom Typ der Hydrotalcite, die man als Feststoffe in die Reaktionsmischung eindosiert. Die Insertion von Alkylenoxiden in die Carbonylester-bindung ist hingegen wesentlich schwieriger und gelingt nur unter Einsatz besonderer Katalysatoren.

Aus den beiden Patentschriften **EP-B1 0 339 425** und **EP-B1 0 523 089** (Henkel) ist die Verwendung von calcinierten bzw. mit Fettsäuren modifizierten Hydrotalciten für die Ethoxylierung von Fettsäureestern bekannt. In der Offenlegungsschrift **DE-A1 44 46 064** (Lion) wird vorgeschlagen, die Ethoxylierung von Methylestern in Gegenwart von Mischmetalloxiden durchzuführen, deren Oberfläche mit Metallhydroxiden bzw. Metallalkoxiden modifiziert worden ist. Diese Verfahren weisen jedoch eine Reihe von Nachteilen auf: Der Einsatz heterogener, d.h. im Reaktionsgemisch nicht löslicher Katalysatoren ist technisch aufwendiger, da der Feststoff nicht wie eine Flüssigkeit über eine automatische Dosiervorrichtung eingebracht werden kann, sondern üblicherweise von Hand in der Reaktor geschaufelt werden muß. Auch die Abtrennung bereitet Probleme, da der Katalysator in der Regel so feinteilig ist, daß die Filtration nur über spezielle Filterkerzen gelingt. Ein Verbleiben des Katalysators im Reaktionsendprodukt ist indes auch nicht möglich, da es sonst zu Austrübungen und zu Sedimentation kommen kann. Auch aus anwendungstechnischer Sicht sind die mit heterogenen Katalysatoren erzielten Ergebnisse nicht immer zufriedenstellend: So läuft die Reaktion zwar in der Regel sehr rasch ab, es werden jedoch üblicherweise Produkte erhalten, die eine deutlich unterhalb des theoretischen Wertes liegende Hydroxylzahl aufweisen. In der Konsequenz werden Produkte erhalten, deren Trübungspunkte unerwünscht hoch liegen. Da das Optimum der Waschleistung nichtionischer Tenside jedoch bekanntlich erst oberhalb ihres Trübungspunktes erreicht wird, besteht ein Bedürfnis nach alkoxylierten Alkylestern, deren Trübungspunkt so niedrig als möglich liegt.

Die Aufgabe der Erfindung hat somit darin bestanden, neue Katalysatoren für die Alkoxylierung von Fettsäurealkylestern zur Verfügung zu stellen, die frei von den geschilderten Nachteilen sind und sich dabei einerseits im Reaktionsprodukt lösen und andererseits Produkte mit verbesserter Hydroxylzahl und niedrigerem Trübungspunkt liefern.

### Beschreibung der Erfindung

Gegenstand der Erfindung ist ein Verfahren zur Herstellung von alkoxylierten Fettsäurealkylestern der Formel **(I)**, 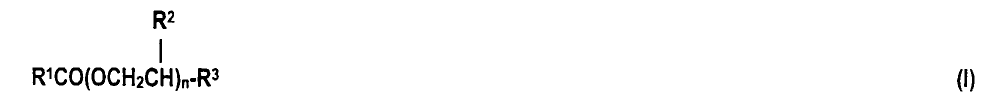 in der R¹CO für einen linearen oder verzweigten Acylrest mit 6 bis 22 Kohlenstoffatomen und 0 und/oder 1, 2 oder 3 Doppelbindungen, R² für Wasserstoff oder eine Methylgruppe, R³ für einen linearen oder verzweigten Alkylrest mit 1 bis 22 Kohlenstoffatomen und n für Zahlen von durchschnittlich 1 bis 20 steht mittels Alkoxylierung von Fettsäurealkylestern, bei dem man als Katalysatoren Mischungen von
(a) Alkali- und/oder Erdalkalihydroxiden und/oder -alkoholaten und
(b) Alkylenglycolen
einsetzt.

Während Alkalihydroxide alleine die Alkoxylierung von Alkylestem nicht katalysieren und die Verwendung von Alkalialkoholat-Katalysatoren zu Produkten mit sehr hohem Trübungspunkt führt, wurde überraschenderweise gefunden, daß der Zusatz von Alkylenglycolen zu diesen Katalysatoren die Aktivität und/oder Selektivität signifikant verbessert. Unter Einsatz des erfindungsgemäßen Systems aus homogenem Katalysator und Co-Katalysator werden jedoch überraschenderweise alkoxylierte Alkylester erhalten, die eine Hydroxylzahl in der Nähe der Theorie aufweisen und sich durch einen sehr niedrigen Trübungspunkt auszeichnen.

### Fettsäurealkylester

Die als Einsatzstoffe in Betracht kommenden Fettsäurealkylester leiten sich von gesättigten und/oder ungesättigten Fettsäuren mit 6 bis 22, vorzugsweise 12 bis 18 Kohlenstoffatomen und Alkoholen mit 1 bis 22, vorzugsweise 1 bis 4 Kohlenstoffatomen ab. Typische Beispiele sind Methyl-, Ethyl-, Propyl-, Butyl- und/oder Stearylester von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technischen Mischungen, die z.B. bei der Druckspaltung von natürlichen Fetten und Ölen oder bei der Reduktion von Aldehyden aus der Roelen'schen Oxosynthese anfallen. Vorzugsweise werden als Einsatzstoffe Kokos- und/oder Talgfettsäuremethylester eingesetzt.

### Katalysatoren

Als Katalysatorkomponente (a) kommen Alkalihydroxide, Erdalkalihydroxide, Alkalialkoholate und/oder Erdalkalialkoholate in Betracht. Typische Beispiele sind Natriumhydroxid, Kaliumhydroxid, Magnesiumhydroxid, Calciumhydroxid, Bariumhydroxid, Natriummethylat, Kalium-tert.butylat, Calciumethylat und/oder Magnesiumethylat. Vorzugsweise werden Kaliumhydroxid und/oder Natriummethylat eingesetzt. Die Einsatzmenge der Katalysatoren (a) beträgt üblicherweise 0,1 bis 1,5 und insbesondere 0,4 bis 1 Gew.-% - bezogen auf die Endprodukte.

Als Katalysatorkomponente (b) - also als Co-Katalyator - kommen Alkylenglycole mit 2 bis 6 Kohlenstoffatomen in Frage, wie beispielsweise Ethylenglycol, Diethylenglycol, Propylenglycol und/oder Dipropylenglycol. Die Einsatzmenge der Co-Katalysatoren beträgt üblicherweise 0,5 bis 5 und vorzugsweise 1 bis 4 Gew.-% - ebenfalls bezogen auf die Endprodukte.

### Alkoxylierung

Die Alkoxylierung kann in an sich bekannter Weise durchgeführt werden. Hierzu legt man üblicherweise den Alkylester in einem Druckreaktor mit Rührer vor und setzt den homogenen Katalysator beispielsweise als Lösung in Wasser, vorzugsweise aber in Methanol zu. Es hat sich als vorteilhaft erwiesen, den Autoklav vor der Reaktion gründlich mit Stickstoff zu spülen, um alle Spuren von Luftsauerstoff zu entfernen und etwa als Lösungsmittel eingesetztes Methanol durch Evakuieren zu entfernen. Danach wird der Druckbehälter aufgeheizt, wobei die Alkoxylierung vorzugsweise bei Temperaturen im Bereich von 140 bis 180 und insbesondere von 160 bis 170°C durchgeführt wird. Das Alkylenoxid, bei dem es sich um Ethylenoxid, Propylenoxid oder Mischungen von beiden handeln kann, wird über einen Heber in den Reaktor eingepreßt, wobei der autogene Druck bis auf etwa 5 bar ansteigen kann. Vorzugsweise werden pro Mol Alkylester durchschnittlich 1 bis 20 und insbesondere 8 bis 15 Mol Alkylenoxid, vorzugsweise Ethylenoxid eingesetzt. Die Anlagerung des Alkylenoxids erfolgt dabei statistisch, d.h. bei der Insertion handelt es sich um keine hochselektive Reaktion bei der 1 Mol Fettsäurealkylester mit exakt n Mol Alkylenoxid reagiert. Vielmehr wird ein komplexes Gemisch unterschiedlich hoch alkoxylierter Ester erhalten. Das Ende der Reaktion ist daran zu erkennen, daß der Druck im Reaktor auf etwa 0,5 bar abfällt. Aus Sicherheitsgründen empfiehlt es sich, die Mischung noch weitere 30 min zu rühren, eher der Reaktor abgekühlt und entspannt wird. Der alkalische Katalysator kann, falls gewünscht, durch Zugabe von Säuren, beispielsweise Phosphorsäure, Essigsäure, Milchsäure oder dergleichen neutralisiert werden.

### Gewerbliche Anwendbarkeit

Die nach dem erfindungsgemäßen Verfahren erhältlichen alkoxylierten Fettsäurealkylester weisen Hydroxylzahlen in der Nähe der Theorie auf und zeichnen sich durch vergleichsweise niedrige Trübungspunkte auf. Gegenüber vergleichbaren Produkten des Stands der Technik, die unter Verwendung anderer Katalysatoren hergestellt worden sind, wird das optimale Waschvermögen somit bei niedrigeren Temperaturen erreicht.

### Beispiele

**Allgemeine Herstellvorschrift.** In einem 1-I-Rührautoklaven wurden 256 g (1 mol) technischer C_{12/18}-Kokosfettsäuremethylester vorgelegt und mit der vorgesehenen Menge Katalysator in methanolischer Lösung (ca. 30 Gew.-%ig) versetzt. Der Autoklav wurde verschlossen und dreimal abwechselnd mit Stickstoff gespült und evakuiert, um die Anwesenheit von Luftsauerstoff auszuschließen und das Lösungsmethanol abzutrennen. Anschließend wurde die Reaktionsmischung unter Stickstoffabdeckung auf 165°C erhitzt und portionsweise 484 g (11 mol) Ethylenoxid eindosiert, wobei der autogene Druck zunächst bis auf 3,5 bar anstieg. Die Reaktion wurde fortgeführt, bis der Druck auf 0,5 bar abgesunken war. Nach weiteren 30 min Nachrührzeit wurde der Druckreaktor abgekühlt und entspannt. Die Ergebnisse der Versuche sind in Tabelle 1 zusammengefaßt. Die Mengenangaben bei den Katalysatoren beziehen sich auf das Endprodukt. Die theoretische Hydroxylzahl betrug 23,2.

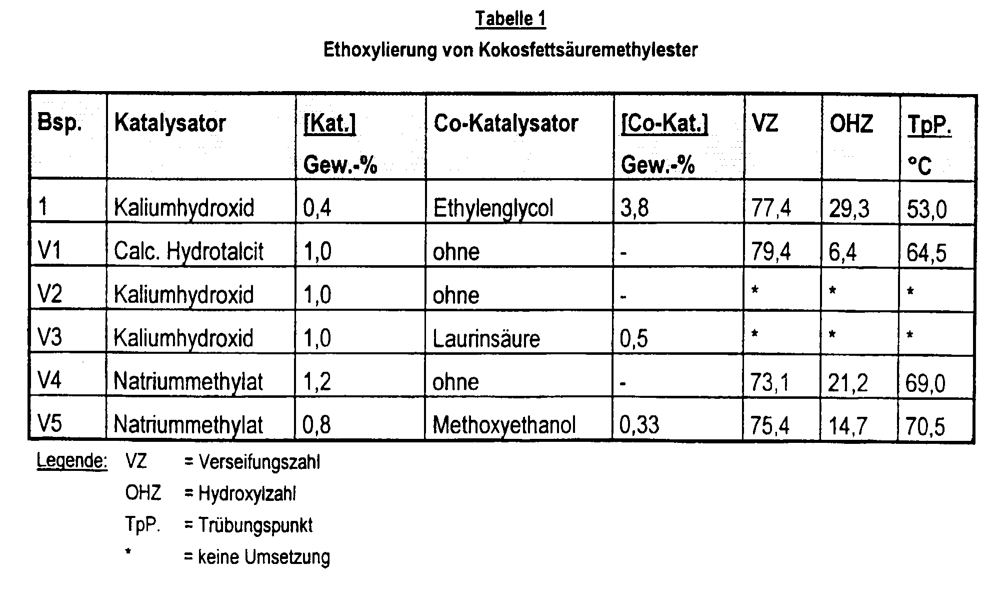

## Patentansprüche

1. Verfahren zur Herstellung von alkoxylierten Fettsäurealkylestem der Formel **(I)**, 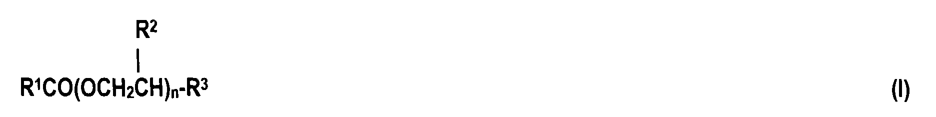 in der R¹CO für einen linearen oder verzweigten Acylrest mit 6 bis 22 Kohlenstoffatomen und 0 und/oder 1, 2 oder 3 Doppelbindungen, R² für Wasserstoff oder eine Methylgruppe, R³ für einen linearen oder verzweigten Alkylrest mit 1 bis 22 Kohlenstoffatomen und n für Zahlen von durchschnittlich 1 bis 20 steht mittels Alkoxylierung von Fettsäurealkylestern, bei dem man als Katalysatoren Mischungen von
(a) Alkali- und/oder Erdalkalihydroxiden und/oder Alkalialkoholaten und
(b) Alkylenglycolen
einsetzt.

2. Verfahren nach Anspruch 1, **dadurch gekennzeichnet**, daß man ethoxylierte Fettsäurealkylester der Formel **(I)** herstellt, bei der R¹CO für einen linearen, gesättigten Acylrest mit 12 bis 18 Kohlenstoffatomen, R² für und R³ für jeweils eine Methylgruppe und n für Zahlen von 8 bis 15 steht.

3. Verfahren nach den Ansprüchen 1 und 2, **dadurch gekennzeichnet**, daß man als Katalysatorkomponente (a) Kaliumhydroxid und/oder Natriummethylat einsetzt.

4. Verfahren nach den Ansprüchen 1 bis 3, **dadurch gekennzeichnet**, daß man als Katalysatorkomponente (b) Ethylenglycol einsetzt.

5. Verfahren nach den Ansprüchen 1 bis 4, **dadurch gekennzeichnet**, daß man die Katalysatorkomponente (a) in Mengen von 0,1 bis 1,5 Gew.-% - bezogen auf die Endprodukte - einsetzt.

6. Verfahren nach den Ansprüchen 1 bis 5, **dadurch gekennzeichnet**, daß man die Katalysatorkomponente (b) in Mengen von 0,5 bis 5 Gew.-% - bezogen auf die Endprodukte - einsetzt.

7. Verfahren nach den Ansprüchen 1 bis 6, **dadurch gekennzeichnet**, daß man die Alkoxylierung in an sich bekannter Weise unter autogenem Druck bei Temperaturen im Bereich von 140 bis 180°C durchführt.

## Claims

1. A process for the production of alkoxylated fatty acid alkyl esters corresponding to formula **(I)**: 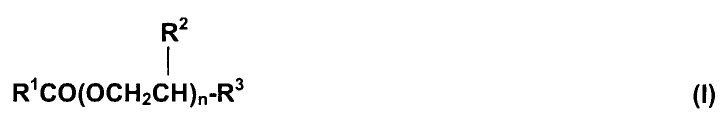 where R¹CO is a linear or branched acyl group containing 6 to 22 carbon atoms and 0 and/or 1, 2 or 3 double bonds, R² is hydrogen or a methyl group, R³ is a linear or branched alkyl group containing 1 to 22 carbon atoms and n is a number of - on average - 1 to 20,
by alkoxylation of fatty acids, in which mixtures of
(a) alkali metal and/or alkaline earth metal hydroxides and/or alcoholates and
(b) alkylene glycols
are used as catalysts.

2. A process as claimed in claim 1, characterized in that ethoxylated fatty acid alkyl esters corresponding to formula **(I)**, in which R¹CO is a linear saturated acyl group containing 12 to 18 carbon atoms, R² and R³ are methyl groups and n is a number of 8 to 15, are produced.

3. A process as claimed in claims 1 and 2, characterized in that potassium hydroxide and/or sodium methylate is/are used as catalyst component (a).

4. A process as claimed in claims 1 to 3, characterized in that ethylene glycol is used as catalyst component (b).

5. A process as claimed in claims 1 to 4, characterized in that catalyst component (a) is used in quantities of 0.1 to 1.5% by weight, based on the end products.

6. A process as claimed in claims 1 to 5, characterized in that catalyst component (b) is used in quantities of 0.5 to 5% by weight, based on the end products.

7. A process as claimed in claims 1 to 6, characterized in that the alkoxylation is carried out in known manner under autogenous pressure at temperatures in the range from 140 to 180°C.

## Revendications

1. Procédé de préparation d'alkylesters d'acide gras alcoxylés de formule (I) 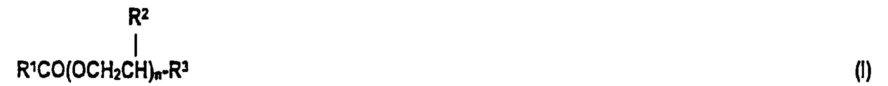 dans laquelle R¹CO représente un reste acyle linéaire ou ramifié ayant de 6 à 22 atomes de carbone et possède O et/ou 1, 2 ou 3 doubles liaisons, R² représente de l'hydrogène ou un groupe méthyle, R³ représente un radical alkyle linéaire ou ramifié ayant de 1 à 22 atomes de carbone, et n représente un nombre de, en moyenne, 1 à 20,
par l'alkoxylation d'alkylesters d'acide gras, procédé dans lequel on met en oeuvre comme catalyseurs des mélanges :
a) d'hydroxydes de métal alcalin et/ou de métal alcalino-terreux et/ou des alcoolates de métal alcalin, et
b) d'alkylèneglycols.

2. Procédé selon la revendication 1,
caractérisé en ce qu'
on prépare des alkylesters d'acide gras éthoxylés de formule (I), dans laquelle R¹CO représente un reste acyle linéaire saturé, ayant de 12 à 18 atomes de carbone, R² et R³ représentent respectivement un groupe méthyle et n représente un nombre allant de 8 à 15.

3. Procédé selon les revendications 1 et 2,
caractérisé en ce qu'
on met en oeuvre comme composant de catalyseur a) de l'hydroxyde de potassium et/ou du méthylate de sodium.

4. Procédé selon les revendications 1 à 3,
caractérisé en ce qu'
on met en oeuvre comme composant de catalyseur b) de l'éthylèneglycol.

5. Procédé selon les revendications 1 à 4,
caractérisé en ce qu'
on met en oeuvre le composant de catalyseur a) en quantités allant de 0,1 à 1,5 % en poids rapporté aux produits finaux.

6. Procédé selon les revendications 1 à 5,
caractérisé en ce qu'
on met en oeuvre le composant de catalyseur b) en quantités allant de 0,5 à 5 % en poids, rapporté aux produits finaux.

7. Procédé selon les revendications 1 à 6,
caractérisé en ce qu'
on effectue l'alkoxylation d'une manière connue en soi, sous pression autogène et à des températures dans la plage de 140 à 180°C.
